# EUROPEAN PATENT SPECIFICATION

(11) **EP 4 321 605 B1**
(45) Date of publication and mention of the grant of the patent: **28.10.2026**
(21) Application number: 21945199.4
(22) Date of filing: 11.06.2021
(51) Int. Cl.: C12M 1/00

(54) **CELL CULTURE DEVICE**
ZELLKULTURVORRICHTUNG
DISPOSITIF DE CULTURE CELLULAIRE

(43) Date of publication of application: 14.02.2024
(73) Proprietor: Fullstem Co., Ltd., Naha-shi, Okinawa 900-0005 (JP)
(72) Inventor: CHIBA, Shunmei, Naha-shi, Okinawa 900-0005 (JP)
(74) Representative: Germain Maureau
(86) International application number: PCT/JP2021/022317
(87) International publication number: WO 2022/259527

(56) References cited:
- EP-A1- 3 572 144
- EP-A2- 1 333 085
- WO-A1-2019/160000
- JP-A- 2004 313 007
- US-A1- 2021 155 887

## Description

### TECHNICAL FIELD

The present invention relates to a cell culture device capable of stirring a medium, supplying nutrients to cells, and oxygenating cells with a simple operation, and capable of culturing a large number of cells.

### BACKGROUND ART

Pluripotent stem cells (embryonic stem cells, induced pluripotent stem (iPS) cells) have been recognized as an important cell source of regenerative medicine due to their infinite proliferative capacity and pluripotency. For example, treatment of liver cirrhosis, blood disease, myocardial infarction, construction of blood vessels, regeneration of bone and cornea, securing of skin for transplantation, and the like are considered as regenerative medicine using stem cells. In regenerative medicine, intended cells and organs are grown from stem cells and the like in a culture dish and transplanted to a human. Recently, angiogenesis has been performed from bone marrow-derived stem cells, resulting in successful treatment in angina pectoris, myocardial infarction, and the like.

In recent years, a cell culture device that cultures a large number of stem cells efficiently under an artificial environment has been required in the fields of pharmaceutical production, gene therapy, regenerative medicine, immunotherapy, and the like.

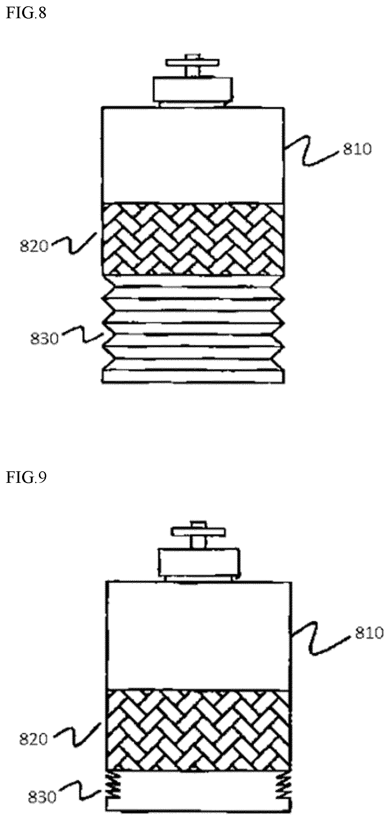

FIG. 8 illustrates a known cell culture device. As shown in FIG. 8, the known cell culture device includes a first chamber 810, a porous scaffold 820, and a second chamber 830. The second chamber 830 has a bellow shape that can be compressed and depressurized. In FIG. 8, the second chamber 830 is in an uncompressed shape and is filled with a culture solution. The scaffold 820 is indirectly exposed to a gas environment, causing cells to undergo oxygenation. In FIG. 9, the second chamber 830 is compressed, pushing the culture solution up into the first chamber 810. The scaffold 820 is immersed in the culture solution, and the cells are supplied with nutrients. The culture solution moves downward and fills the second chamber 830 when the second chamber 830 is uncompressed again as in FIG. 8.

However, according to this cell culture device, the second chamber 830 has a bellow shape, and the cells that have moved to the second chamber 830 enter protrusions protruding to the outside of the bellow shape. Such cells are less likely to be affected by the culture solution moving up and down when the second chamber 830 is compressed and uncompressed, which may the oxygenation difficult. In addition, the second chamber 830, particularly a central portion of the second chamber 830, cannot return the culture solution completely to the first chamber 810, even in the compressed bellow shape, because of the structure of the second chamber 830. Thus, in order to seed cells on the porous scaffold 820, a cell suspension is placed in the first chamber 810 and kept static, or is caused to undergo cell adhesion by compressing and uncompressing the bellow shape for cell adhesion. However, there would be a cell suspension that is kept from cell adhesion due to a dead space and dead volume generated, which is inefficient.

### CITATION LIST

### PATENT DOCUMENTS

PATENT DOCUMENT 1: Japanese Patent No. 4430317

EP3572144 describes a cell-culturing device, comprising a semipermeable membrane, including a holding body with a low water absorption property having a lattice structure and having a semipermeable property in a liquid phase.

WO2019/160000 A1 discloses a cell culture device.

### SUMMARY OF THE INVENTION

### TECHNICAL PROBLEM

The present invention has been made in view of such problems, and it is an object of the present invention to provide a cell culture device that enables sufficient nutrient supply and oxygenation to cells with a simple structure and enables mass culture of appropriate stem cells.

### SOLUTION TO THE PROBLEM

A cell culture device of the present invention includes a cell culture vessel that is a substantially cylindrical body having, at a lower end, a bottom that is flat, and having a hollow space to be filled with a medium for culturing cells; a dish-shaped body having, in a periphery, a plurality of first magnetic bodies each made of a magnet or a ferromagnetic material at equal intervals, the dish-shaped body having a substantially disk shape with a diameter slightly smaller than a diameter of the substantially cylindrical body of the cell culture vessel so as to be disposed horizontally in the hollow space of the cell culture vessel; and an annular body having a plurality of second magnetic bodies each made of a magnet corresponding to an associated one of the first magnetic bodies of the dish-shaped body to attract the first magnetic bodies of the dish-shaped body by a magnetic force, the annular body having a substantially ring shape and positioned outside the cell culture vessel to have the cell culture vessel located inside a ring of the cell culture vessel, the annular body being configured to move upward to cause the dish-shaped body to move upward in conjunction with the annular body due to the magnetic force, thereby pushing up the medium that fills the hollow space of the cell culture vessel from below, and move downward to cause the dish-shaped body to move downward in conjunction with the annular body due to the magnetic force, thereby making the medium that fills the hollow space of the cell culture vessel fall by gravity.

### ADVANTAGES OF THE INVENTION

The present invention enables sufficient nutrient supply and oxygenation to cells with a simple structure and enables mass culture of appropriate stem cells.

### BRIEF DESCRIPTION OF THE DRAWINGS

FIG. 1 is a diagram illustrating the appearance of a cell culture device according to the present embodiment.
FIG. 2 is a diagram illustrating in detail a vicinity of a dish-shaped body of the cell culture device according to the present embodiment.
FIG. 3 is a diagram illustrating in detail a vicinity of a bottom of a cell culture vessel of the cell culture device according to the present embodiment.
FIG. 4 is a diagram illustrating a storage provided below the bottom of the cell culture vessel according to the present embodiment.
FIGS. 5A and 5B are plan views illustrating an annular body of the cell culture vessel according to the present embodiment. FIG. 5A shows a state in which a second magnetic body slides radially outward. FIG. 5B shows a state in which the second magnetic body slides radially inward.
FIG. 6 is an enlarged view illustrating a state in which the second magnetic body slides radially inward.
FIG. 7 is an enlarged view illustrating a state in which the second magnetic body slides radially outward.
FIG. 8 is a diagram illustrating a known cell culture device in which a bellow-shaped chamber is uncompressed.
FIG. 9 is a diagram illustrating the known cell culture device in which the bellow-shaped chamber is compressed.

### DESCRIPTION OF EMBODIMENT

Embodiments of the present invention will be described in detail below while referring to the accompanying drawings. The embodiments are only intended to facilitate the understanding of the principles of the present invention, and the scope of the present invention is not limited to the following embodiments. Other embodiments where a person skilled in the art can appropriately replace configurations of the embodiments below are included within the scope of the present invention.

### (1) First Embodiment

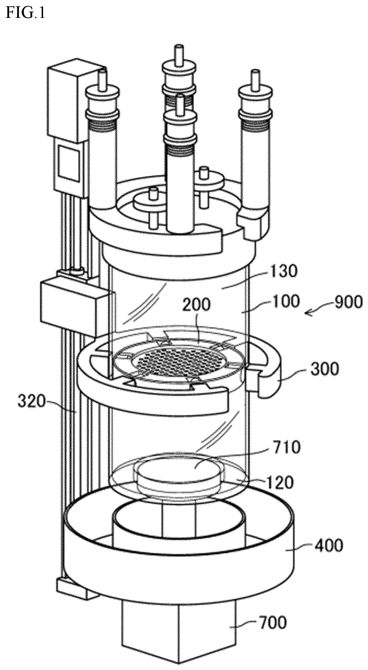

As shown in FIG. 1, a cell culture device 900 according to the present embodiment includes a cell culture vessel 100 which is a substantially cylindrical body, a dish-shaped body 200 having a substantially disk shape, an annular body 300 having a substantially ring shape, and a storage 400 that stores the annular body 300. The substantially cylindrical body is a columnar body whose cross-sectional shape on a horizontal plane includes not only a circle but also an ellipse, an oval shape, and so on. The substantially disk shape is a shape including not only a circle but also an ellipse, an oval shape, and so on in plan view. The substantially ring shape is a shape including not only circular ring shape but also an elliptical ring shape and an oval ring shape in plan view.

The cell culture vessel 100 has an opening (not shown) at its upper end and a flat bottom 120 at its lower end. The cell culture vessel 100 includes a hollow space 130 to be filled with a scaffold for culturing cells. The scaffold, a medium, and the cells to be cultured can be inserted from the opening at the upper end of the cell culture vessel 100.

The cells to be cultured are not limited and may be cells that are less likely to be damaged and cells that are likely to be damaged. The cells to be cultured are preferably cells that are likely to be damaged, and more preferably, cells such as human embryonic stem (ES) cells, iPS cells, and somatic stem cells. Preferably, the somatic stem cells are, for example, adipose-derived or bone marrow-derived mesenchymal stem cells. The medium to be used is a liquid medium and includes, for example, glucose, etc.

The dimension of the cell culture vessel 100 is not limited. For example, the volume can be 250 ml (100 mm in diameter, 50 mm in height, 100 mm at bottom), 500 ml (100 mm in diameter, 100 mm in height, 100 mm at bottom), 1000 ml (100 mm in diameter, 200 mm in height, 100 mm at bottom), or the like.

The cell culture vessel 100 is a vessel, to the inner wall of which cells are less likely to adhere, and is made, for example, of plastic with low adhesiveness, such as polyethylene terephthalate, polypropylene, polyethylene, polycarbonate, and polystyrene, plastic subjected to hydrophobic surface treatment, such as fluorine treatment or silicon treatment, or made of glass.

The cells cultured in the cell culture vessel 100 are vibrated up and down by a vibrator 710 of a vibration device 700. The vibration device 700 is not limited, and is, for example, a vortex.

The scaffold that fills the space 130 of the cell culture vessel 100 is, for example, a three-dimensional porous scaffold. The three-dimensional porous scaffold has, for example, an average porosity of 50% to 90%, preferably 80% to 90%, and an average pore size of 10 µm to 800 µm, preferably 200 µm to 400 µm. The scaffold is a single scaffold or a scaffold made of an aggregate of a plurality of small pieces supporting cells. A culture area of the scaffold is not limited, and is, for example, 800 cm² to 900000 cm², preferably 800 cm² to 45000 cm². In the case of the scaffold made of an aggregate of a plurality of small pieces supporting cells, the scaffold may be, for example, a fiber assembly (nonwoven fabric, woven fabric, knitted fabric, and the like) made of fibers of a thermoplastic resin, or an assembly of sheets prepared by kneading a thermoplastic resin in advance and then molding the thermoplastic resin.

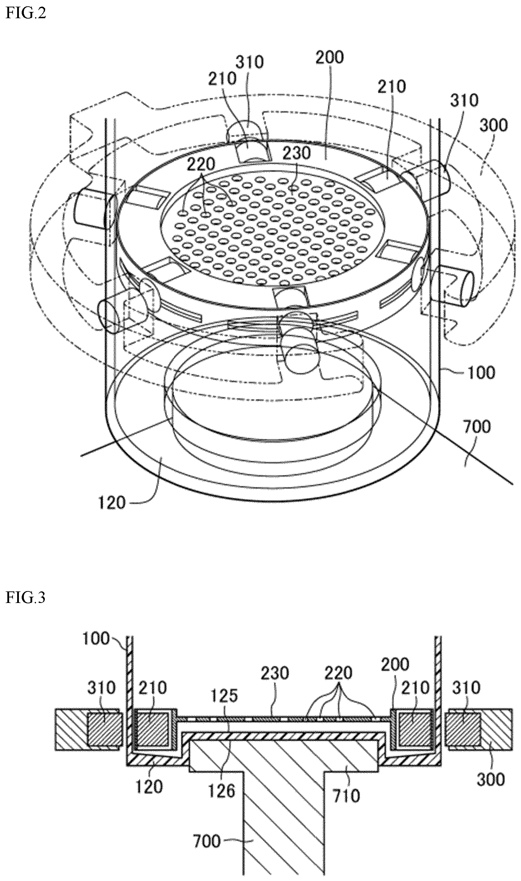

As shown in FIG. 2, the dish-shaped body 200 has a plurality of first magnetic bodies 210 at equal intervals in a periphery of the dish-shaped body 200. The dish-shaped body 200 has, for example, a thickness of 15 mm, an outer diameter of 95 mm, and an inner diameter of 68 mm. The first magnetic bodies 210 are a metal of ferromagnetic material in consideration of an influence on cultured cells. For example, the first magnetic bodies 210 are a metal containing iron, cobalt, and nickel as main components. In a case of using magnets as the first magnetic bodies 210, the magnets preferably have a low magnetic force in order to reduce the influence on the cultured cells.

The shape of the first magnetic bodies 210 is not limited. The first magnetic bodies 210 may be, for example, a cylindrical body having a diameter of 10 mm and a height of 10 mm. In the present embodiment, six first magnetic bodies 210 are provided in the periphery of the dish-shaped body 200 having a substantially disk shape at equal intervals, each 60 degrees. The magnetic force of the first magnetic bodies 210 is 0 gauss in the case of iron. The dish-shaped body 200 is placed horizontally in the hollow space of the cell culture vessel 100. The dish-shaped body 200 having a substantially disk shape has a diameter slightly smaller than the diameter of the substantially cylindrical body of the cell culture vessel 100. Specifically, it is possible to set the diameter D₂ of the dish-shaped body 200 having a substantially disk shape to be 0.90D₁ to 0.998D₁, where D₁ is the inner diameter of the substantially cylindrical body of the cell culture vessel 100. This means that the dish-shaped body 200 having a substantially disk shape does not come into contact with the inner surface of the cell culture vessel 100. It is possible to provide wheels on the outer periphery of the dish-shaped body 200 having a substantially disk shape at equal intervals, and bring the wheels into contact with the inner surface of the cell culture vessel 100.

The annular body 300 having a substantially ring shape is positioned outside the cell culture vessel 100, and the cell culture vessel 100 is positioned inside the ring. The thickness of the annular body 300 is, for example, the same as the thickness of the dish-shaped body 200, and is 15 mm. The annular body 300 having a substantially ring shape has a plurality of second magnetic bodies 310 made of magnets so as to correspond to the respective first magnetic bodies 210 of the dish-shaped body 200. The shape of the second magnetic bodies 310 is not limited. The second magnetic bodies 310 can be, for example, a cylindrical body having a diameter of 10 mm and a height of 16 mm. The magnetic force of the second magnetic bodies 310 is, for example, 4500 gauss to 4800 gauss in the case of a permanent magnet. A distance between an end on one side of the first magnetic body 210 of the dish-shaped body 200 and an end on the other side of the second magnetic body 310 of the annular body 300 is, for example, 0.1 mm to 10.0 mm. Since the second magnetic bodies 310 of the annular body 300 correspond to the respective first magnetic bodies 210 of the dish-shaped body 200, the height (that is, the position in the vertical direction) of the annular body 300 coincides with the height of the dish-shaped body 200 when the annular body 300 moves up and down along the cell culture vessel 100. The state in which the height of the annular body 300 and the height of the dish-shaped body 200 coincide with each other can also be expressed as the state in which the annular body 300 and the dish-shaped body 200 are aligned at the same vertical height.

A plurality of through holes 220 are provided in a central portion of the dish-shaped body 200. The hole diameter of the through hole 220 is not limited and can be, for example, 1 mm to 5 mm.

The annular body 300 is supported by a lift device 320 and moves up and down. Since the annular body 300 moves up and down, the dish-shaped body 200 also moves up and down due to the magnetic force in conjunction with the annular body 300. This configuration makes it possible to stir the medium and to oxygenate cells and supply nutrients to the cells. The motion of the up-down movement of the annular body 300 is, for example, a simple harmonic motion or a substantially simple harmonic motion. The cycle of the up-down movement of the annular body 300 can be, for example, 0.5 times/hour to 12.0 times/hour. The substantially simple harmonic motion is, for example, a repeated motion including moving from a lower side to an upper side and stopping there for a certain period of time, and subsequently moving from the upper side to the lower side and stopping there for a certain period of time, and again moving from the lower side to the upper side. The stroke length of the up-down movement of the annular body 300 is greater than the height of the cell culture vessel 100. Specifically, the annular body 300 can move up and down in a range from around an upper part of the cell culture vessel 100 to further below the bottom 120 of the cell culture vessel 100, and the stroke length of the up-down movement of the annular body 300 can be, for example, 80 mm to 260 mm. The moving speed of the annular body 300 in an upward direction or a downward direction may be, for example, 0.5 mm/sec to 10.0 mm/sec.

As shown in FIG. 3, the cell culture vessel 100 has a bottom protrusion 125 which protrudes upward at a central portion of the bottom 120 and has a space inside. In other words, the cell culture vessel 100 has a bottom recess 126 having a shape corresponding to the bottom protrusion 125, at the central portion of the bottom 120. For example, in a case where the diameter of the bottom 120 of the cell culture vessel 100 is 100 mm, the depth of the bottom recess 126 is 8 mm, and the diameter of the inside of the bottom recess 126 is 60 mm. The vibrator 710 of the vibration device 700 is fitted into the bottom recess 126 without any gap.

The dish-shaped body 200 includes a dish recess 230 in which the bottom protrusion 125 of the cell culture vessel 100 is fitted, at a central portion of a lower surface of the dish-shaped body 200. Although in FIG. 3 there is a gap between the bottom protrusion 125 of the cell culture vessel 100 and the dish recess 230 of the dish-shaped body 200, the bottom protrusion 125 may be fitted into the dish recess 230 without any gap.

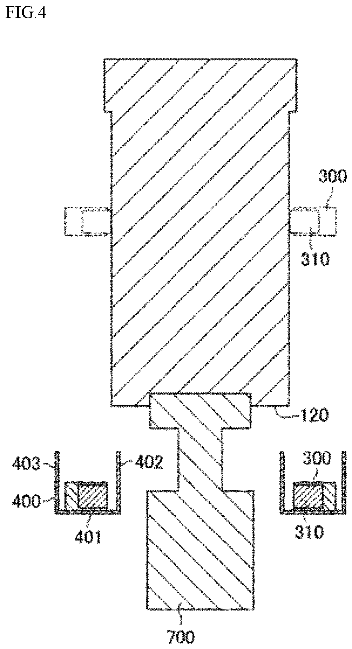

As shown in FIG. 4, a storage 400 is provided below the bottom 120 of the cell culture vessel 100. The annular body 300 moves downward below the bottom 120 of the cell culture vessel 100, and the annular body 300 is stored in the storage 400. The storage 400 has a substantially ring shape in plan view so that the annular body 300 having a substantially ring shape is stored therein.

The storage 400 is made of a ferromagnetic material, specifically made of a metal containing iron, cobalt, and nickel as main components. Since the storage 400 is made of the ferromagnetic material, the storage 400 reduces the action, to the outside, of the magnetic force of the second magnetic bodies 310 inside the annular body 300 when the annular body 300 is stored in the storage 400. It is therefore possible to reduce the influence of the magnetic force of the second magnetic bodies 310 on the cultured cells.

The storage 400 includes a bottom peripheral wall 401 circumferentially surrounding the bottom of the annular body 300, an outer peripheral wall 403 provided outside the bottom peripheral wall 401 and circumferentially surrounding the outside of the annular body 300, and an inner peripheral wall 402 provided inside the bottom peripheral wall 401 and circumferentially surrounding the inside of the annular body 300. The thicknesses of the bottom peripheral wall 401, the inner peripheral wall 402, and the outer peripheral wall 403 are determined from the viewpoint of shielding the magnetic force of the second magnetic bodies 310 to the outside. The thicknesses are not limited, and, for example, 0.1 mm or more and 2 mm or less, preferably 0.5 mm or more and 1.5 mm or less, and more preferably 0.8 mm or more and 1.2 mm or less.

A distance between the bottom peripheral wall 401 of the storage 400 and the bottom 120 of the cell culture vessel 100 is not limited, but is, for example, 30 mm or more or 200 mm or less, preferably 40 mm or more and 150 mm or less, and more preferably 50 mm or more and 100 mm or less.

The inner peripheral wall 402 and the outer peripheral wall 403 may have the same height or different heights. In one preferred embodiment, The height of each of the inner peripheral wall 402 and the outer peripheral wall 403 is preferably longer than the height of the second magnetic bodies 310 inside the annular body 300. The height is not limited, but is, for example, 12 mm or more and 50 mm or less, preferably 18 mm or more and 40 mm or less, and more preferably 24 mm or more and 30 mm or less.

Next, a mode of use of the cell culture device according to the present embodiment will be described.

First, the dish-shaped body 200 disposed in the hollow space 130 of the cell culture vessel 100 is positioned near a lower part of the cell culture vessel 100. In this case, since the height of the annular body 300 coincides with the height of the dish-shaped body 200, the annular body 300 is also positioned near the lower part of the cell culture vessel 100. The state in which the height of the annular body 300 and the height of the dish-shaped body 200 coincide with each other refers to the state in which the height of a vertically middle portion of the second magnetic body 310 and the height of a vertically middle portion of the first magnetic body 210 coincide with each other. Then, the scaffold, the medium, and the cells to be cultured are placed into the space 130 of the cell culture vessel 100 from the opening (not shown) at the upper end of the cell culture vessel 100.

Next, the annular body 300 positioned near the lower part of the cell culture vessel 100 is moved upward. The second magnetic bodies 310 of the annular body 300 correspond to the respective first magnetic bodies 210 of the dish-shaped body 200. The dish-shaped body 200 also moves upward in conjunction with the annular body 300 due to the magnetic force. As the dish-shaped body 200 moves upward, the scaffold that fills the hollow space 130 of the cell culture vessel 100 and the cells adhering to the scaffold are pushed up from the lower side by the dish-shaped body 200 and moves to the upper side. Although the dish-shaped body 200 moves upward, the medium in the space 130 of the cell culture vessel 100 is not pushed upward but passes through the plurality of through holes 220 provided in the central portion of the dish-shaped body 200. The medium is stirred at this moment.

Next, the annular body 300 brings the dish-shaped body 200 to a position near the upper part of the cell culture vessel 100 and stops the movement. The positioning of the dish-shaped body 200 near the upper part of the cell culture vessel 100 means that the dish-shaped body 200 is at a position separated downward from the upper end of the cell culture vessel 100 by a distance of, for example, 0.3L to 0.7L, where L is the height of the cell culture vessel 100 (the height L is a distance from the opening at the upper end of the cell culture vessel 100 to the bottom 120). **In** the state in which the dish-shaped body 200 is positioned near the upper part of the cell culture vessel 100, the cells, for example, can undergo oxygenation since the medium is not pushed upward.

Next, the annular body 300 is moved downward. The dish-shaped body 200 also moves downward in conjunction with the annular body 300 due to the magnetic force. As the dish-shaped body 200 moves downward, the scaffold and the cells adhering to the scaffold fall by gravity and are immersed in the medium, thus supplying nutrients to the cells.

The annular body 300 continues to move downward to a position near the lower part of the cell culture vessel 100. The dish-shaped body 200 comes into contact with the bottom protrusion 125 of the cell culture vessel 100, and the dish recess 230 of the dish-shaped body 200 is fitted to the bottom protrusion 125 of the cell culture vessel 100 without any gap, where the dish-shaped body 200 stops.

Thereafter, the annular body 300 moves further downward below the bottom 120 of the cell culture vessel 100 and is stored in the storage 400 shown in FIG. 4. When the annular body 300 is stored in the storage 400, the magnetic force action of the second magnetic bodies 310 inside the annular body 300 on the cultured cells is reduced.

After the annular body 300 is stored in the storage 400 for a certain period of time, the annular body 300 stored in the storage 400 is moved upward. When the height of the annular body 300 coincides with the height of the dish-shaped body 200 near the lower part of the cell culture vessel 100 as the annular body 300 continues to move upward, the second magnetic bodies 310 of the annular body 300 correspond to the respective first magnetic bodies 210 of the dish-shaped body 200, causing the dish-shaped body 200 to move upward in conjunction with the annular body 300 due to the magnetic force.

After culturing, the dish recess 230 of the dish-shaped body 200 is fitted to the bottom protrusion 125 of the cell culture vessel 100 without any gap, and the vibrator 710 of the vibration device 700 is fitted into the bottom recess 126 of the cell culture vessel 100 without any gap. In this state, the vibration device 700 is activated to transmit, for example, up-down vibrations from the vibrator 710 to the cultured cells in the cell culture vessel 100. This configuration makes it easier to collect the cultured cells buried deep in the three-dimensional porous scaffold.

In this way, in the first embodiment, it is possible to oxygenate cells and supply nutrients to the cells while reducing the influence of the magnetic force on the cultured cells.

### (2) Second Embodiment

In a case of using a large number of scaffolds, such as a large number of nonwoven fabric sheets, an ascending motion of the dish-shaped body 200 may be inhibited by the weight of the scaffolds. The action of the magnetic force between the first magnetic bodies 210 and the second magnetic bodies needs to be secured appropriately in the case of using a large number of scaffolds as well, in order to perform the ascending motion of the dish-shaped body 200 appropriately. For this purpose, it is necessary to reduce the distance between the first magnetic bodies 210 and the second magnetic bodies 310. However, the annular body 300 stored in the storage 400 may collide with the bottom 120 of the cell culture vessel 100 when ascending if an unexpected external force is applied to the cell culture device 900 in a case where the distance between the first magnetic bodies 210 and the second magnetic bodies 310 is reduced. Thus, the second magnetic bodies 310 in the annular body 300 need to be attracted toward the dish-shaped body 200 when the dish-shaped body 200 moves upward, whereas the second magnetic bodies 310 in the annular body 300 preferably move away from the dish-shaped body 200 when the annular body 300 stored in the storage 400 moves upward.

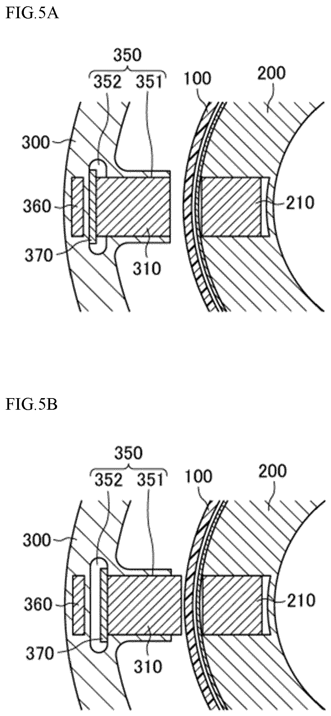

In order to solve the above problem, the cell culture device 900 according to the present embodiment has a characteristic configuration in the annular body 300 in addition to the configuration of the first embodiment described above. That is, in the cell culture device 900 according to the present embodiment, the annular body 300 has a housing 350 in which the second magnetic body 310 is housed as shown in FIGS. 5A and 5B. The housing 350 has a gap therein so that the second magnetic body 310 is slidable in the radial direction of the annular body 300. Thus, the second magnetic body 310 is housed in the housing 350 so as to be slidable in the radial direction.

The housing 350 includes a columnar hollow portion 351 extending in the radial direction of the annular body 300 and a widened portion 352 positioned radially outside the columnar hollow portion 351. The columnar hollow portion 351 and the second magnetic body 310 have the same cross-sectional shape. An inner surface of the columnar hollow portion 351 and an outer surface of the second magnetic body 310 are in close contact with each other. Accordingly, a motion of the second magnetic body 310 other than sliding in the radial direction is restricted. That is, the second magnetic body 310 performs only sliding movement in the radial direction of the annular body 300, and does not move, for example, in a direction perpendicular to the radial direction of the annular body 300.

The widened portion 352 is spatially continuous with the columnar hollow portion 351. As illustrated in FIGS. 5A and 5B, a cross-sectional area perpendicular to the radial direction of the annular body 300 is greater in the widened portion 352 than in the columnar hollow portion 351.

The second magnetic body 310 has a stopper 370 at an outer end in the radial direction of the annular body 300. As illustrated in FIGS. 5A and 5B, a cross-sectional area perpendicular to the radial direction of the annular body 300 is greater in the stopper 370 than in the columnar hollow portion 351, and is greater in the widened portion 352 than in the stopper 370. The stopper 370 is positioned in the widened portion 352. The length of the stopper 370 in the radial direction of the annular body 300 (that is, the thickness of the stopper 370) is, for example, 1.5 mm.

The annular body 300 includes a third magnetic body 360 on a radially outer side of the housing 350. That is, as shown in FIGS. 5A and 5B, the annular body 300 is provided with the third magnetic body 360 on a radially outer side of the widened portion 352. Similarly to the first magnetic body 210, the third magnetic body 360 is made of a metal of ferromagnetic material. For example, the third magnetic body 360 is a metal containing iron, cobalt, and nickel as main components. The mass of the third magnetic body 360 is smaller than the mass of the first magnetic body 210. In a case of using a magnet as the third magnetic body 360, the magnet preferably has a low magnetic force in order to reduce the influence on the cultured cells.

Unlike the first embodiment, according to the configuration of the second embodiment described above, the annular body 300 includes the housing 350 that houses the second magnetic body 310 slidably, the third magnetic body 360, and the stopper 370. This characteristic configuration can be referred to as an attraction mechanism (because the annular body 300 has a mechanism in which, as described later, the second magnetic body 310 is attracted not to the third magnetic body 360 but to the first magnetic body 210 when the height of the annular body 300 and the height of the dish-shaped body 200 coincide with each other).

Next, a mode of use of the cell culture device according to the present embodiment will be described.

First, the dish-shaped body 200 disposed in the hollow space 130 of the cell culture vessel 100 is positioned near a lower part of the cell culture vessel 100. The height of the annular body 300 coincides with the height of the dish-shaped body 200 at this moment, and the annular body 300 is also positioned near the lower part of the cell culture vessel 100.

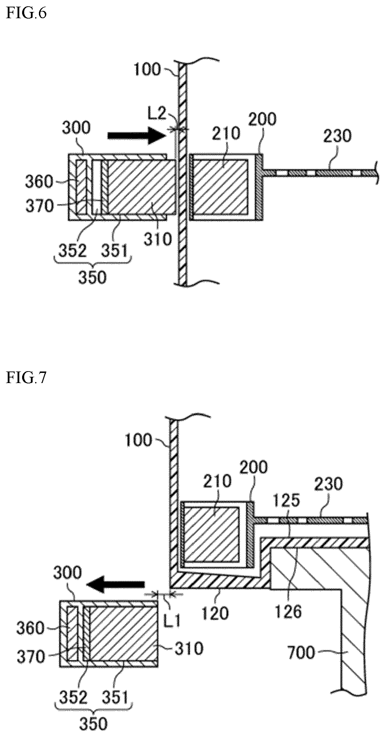

The second magnetic body 310 is a magnet, whereas the first magnetic body 210 and the third magnetic body 360 is a metal of ferromagnetic material. The mass of the third magnetic body 360 is smaller than the mass of the first magnetic body 210. Accordingly, the second magnetic body 310 is attracted not to the third magnetic body 360 but to the first magnetic body 210 when the height of the annular body 300 and the height of the dish-shaped body 200 coincide with each other. As illustrated in FIG. 5A and FIG. 6, the second magnetic body 310 inside the annular body 300 slides in the housing 350 and moves radially inward of the annular body 300. The radially inward refers to a side toward the central portion of the annular body 300. Since the second magnetic body 310 is a magnet and the first magnetic body 210 is a metal of ferromagnetic material, the magnetic force acts on the first magnetic body 210 from the second magnetic body 310. Since the first magnetic body 210 is fixed to the dish-shaped body 200, and the second magnetic body 310 is slidable in the radial direction of the annular body 300, the second magnetic body 310 is attracted to the first magnetic body 210.

As shown in FIG. 5B, the widened portion 352 has a larger cross-sectional area perpendicular to the radial direction of the annular body 300 than the stopper 370, and the stopper 370 has a larger cross-sectional area perpendicular to the radial direction of the annular body 300 than the columnar hollow portion 351. Thus, when the second magnetic body 310 is attracted to the first magnetic body 210, the stopper 370 abuts on a radially inner end of the widened portion 352, leaving a gap of a small distance L2 between a radially inner end of the second magnetic body 310 and an outer surface of the cell culture vessel 100. The distance L2 is not limited, and is, for example, 0.05 mm to 0.15 mm, preferably 0.1 mm. As illustrated in FIG. 5B, a gap is generated between a radially outer end of the stopper 370 and the radially inner end of the widened portion 352, and the distance therebetween is, for example, 2.2 mm.

Next, the annular body 300 positioned near the lower part of the cell culture vessel 100 is moved upward. The second magnetic bodies 310 of the annular body 300 correspond to the respective first magnetic bodies 210 of the dish-shaped body 200. The dish-shaped body 200 also moves upward in conjunction with the annular body 300 due to the magnetic force. Since the first magnetic body 210 and the second magnetic body 310 are close to each other at the small distance L2, there is no possibility that the ascending motion of the dish-shaped body 200 is inhibited by the weight of the scaffold even if a large number of scaffolds, such as a large number of nonwoven fabric sheets, are used.

Next, the annular body 300 brings the dish-shaped body 200 to a position near the upper part of the cell culture vessel 100 and stops the movement. In the state in which the dish-shaped body 200 is positioned near the upper part of the cell culture vessel 100, the cells, for example, can undergo oxygenation since the medium is not pushed upward.

Next, the annular body 300 is moved downward. The dish-shaped body 200 also moves downward in conjunction with the annular body 300 due to the magnetic force. The scaffold and the cells adhering to the scaffold fall by gravity and are immersed in the medium, thus supplying nutrients to the cells.

The annular body 300 continues to move downward to a position near the lower part of the cell culture vessel 100. The dish-shaped body 200 comes into contact with the bottom protrusion 125 of the cell culture vessel 100, and the dish recess 230 of the dish-shaped body 200 is fitted to the bottom protrusion 125 of the cell culture vessel 100.

Next, while the dish-shaped body 200 remains fitted to the bottom protrusion 125 of the cell culture vessel 100, the annular body 300 moves further downward below the bottom 120 of the cell culture vessel 100. As the annular body 300 moves downward, the distance between the second magnetic body 310 and the first magnetic body 210 increases, and the second magnetic body 310 that has been attracted to the first magnetic body 210 is finally attracted not to the first magnetic body 210 but to the third magnetic body 360. As illustrated in FIG. 5A and FIG. 7, the second magnetic body 310 slides in the housing 350 and moves radially outward of the annular body 300. The radially outward refers to a side away from the central portion of the annular body 300. Since the second magnetic body 310 is a magnet and the third magnetic body 360 is a metal of ferromagnetic material, the magnetic force acts on the third magnetic body 360 from the second magnetic body 310. Since the third magnetic body 360 is fixed to the annular body 300, and the second magnetic body 310 is slidable in the radial direction of the annular body 300, the second magnetic body 310 is attracted to the third magnetic body 360. The stopper 370 abuts on a radially outer end of the widened portion 352, generating a gap of a distance L1 between the radially inner end of the second magnetic body 310 and the outer surface of the cell culture vessel 100. The distance L1 is not limited, and is, for example, from 2.1 mm to 2.5 mm, preferably 2.3 mm.

Next, as shown in FIG. 4, the annular body 300 is stored in the storage 400 provided below the bottom 120 of the cell culture vessel 100. In the state in which the annular body 300 is stored in the storage 400, the second magnetic body 310 is attracted to the third magnetic body 360, and the stopper 370 is in contact with the radially outer end of the widened portion 352. While the annular body 300 is stored in the storage 400, the storage 400 reduces the action of the magnetic force of the second magnetic body 310 to the outside. It is therefore possible to reduce the influence of the magnetic force on the cultured cells.

Next, the annular body 300 stored in the storage 400 is moved upward. The radially inner end of the second magnetic body 310 is separated from the outer surface of the cell culture vessel 100 by the distance L1. Thus, even if an unexpected external force is applied when the upper end of the annular body 300 comes to be equal in level to the bottom 120 of the cell culture vessel 100, there is very little possibility that the second magnetic body 310 of the annular body 300 abuts on the cell culture vessel 100.

The annular body 300 moves further upward to around a height where the height of the annular body 300 and the height of the dish-shaped body 200 coincide with each other. Then, as shown in FIG. 5B and FIG. 6, the second magnetic body 310 is attracted not to the third magnetic body 360 but to the first magnetic body 210, causing the second magnetic body 310 to slide in the housing 350 and move radially inward of the annular body 300, and causing the first magnetic body 210 and the second magnetic body 310 to approach each other while keeping the distance L2. **In** this state, the dish-shaped body 200 also moves upward in conjunction with the annular body 300.

In this way, in the second embodiment, even in a case where a large number of nonwoven fabric sheets is placed inside, it is possible to ensure an accurate up-down movement of the annular body 300 and to oxygenate cells and supply nutrients to the cells while reducing the influence of the magnetic force on the cultured cells.

### [Examples]

### (Example 1)

An example of cell culture using a cell culture device (the cell culture device according to the first embodiment) having a storage for storing an annular body will be described in this example.

The dish-shaped body disposed in the hollow space of the cell culture vessel was positioned near the lower part of the cell culture vessel. A scaffold, a medium, and cells to be cultured were placed into the space of the cell culture vessel from the opening at the upper end of the cell culture vessel. The cells were 2.0 x 10⁷ adipose-derived mesenchymal stem cells (ADSC). Five hundred sheets of BioNOC II Matrix from CESCO were used as the scaffold (3D culture). The stroke length of the up-down movement of the annular body was 90 mm. That is, the height of the cell culture vessel was 60 mm, and the distance between the bottom of the cell culture vessel and the storage was 30 mm. The moving speed of the annular body in the upward direction or the downward direction was set to be 1.0 mm/sec. The cycle of the up-down movement of the annular body was set to be 1.0 times/hour. The number of days of culture was seven days.

The sugar consumption of the cells was measured using a GlucCell Handy Glucose Monitor (CESCO) to find out that the sugar consumption of the cells increased as the number of days increased, and that the cells were appropriately cultured by using the cell culture device of this example.

On Day 7, as many as 3.0 x 10⁸ ADSCs were collected from within the cell culture vessel. It is difficult to collect the cultured cells buried deep in the three-dimensional porous scaffold efficiently without damaging the cultured cells. However, a large number of cultured cells can be collected from the cell culture vessel by adding, after the culture, an enzyme solution containing trypsin-EDTA and caseinase into the cell culture vessel and performing enzyme treatment on the cultured cells.

Whether the collected ADSCs retained a normal karyotype was analyzed. The collected ADSCs were fixed with Carnoy's solution, and then subjected to simple karyotyping (Q-band) (chromocenter Inc.). The simple karyotyping (Q-band analysis) is a method of identifying each chromosome on the basis of a band pattern that is detected by a chromosome banding technique and characteristic of the chromosome, and analyzing the presence or absence of polyploidy, aneuploidy, translocation, and the like. The result showed that the collected ADSCs retained a normal karyotype. It was clear that the cells cultured by using the cell culture device of this example maintained a normal karyotype.

That is, in Example 1, it was shown that the influence of the magnetic force on the cultured cells was reduced and that the cells were appropriately cultured.

### (Example 2)

An example of cell culture using a cell culture device (the cell culture device according to the second embodiment) having a storage for storing an annular body and including an attraction mechanism will be described in this example.

The dish-shaped body disposed in the hollow space of the cell culture vessel was positioned near the lower part of the cell culture vessel. A scaffold, a medium, and cells to be cultured were placed into the space of the cell culture vessel from the opening at the upper end of the cell culture vessel. The cells were 2.0 x 10⁸ adipose-derived mesenchymal stem cells (ADSCs). Five thousand sheets of BioNOC II Matrix from CESCO were used as the scaffold (3D culture). The stroke length of the up-down movement of the annular body was 90 mm. That is, the height of the cell culture vessel was 60 mm, and the distance between the bottom of the cell culture vessel and the storage was 30 mm. The moving speed of the annular body in the upward direction or the downward direction was set to be 1.0 mm/sec. The cycle of the up-down movement of the annular body was set to be 1.0 times/hour. The number of days of culture was ten days. During the ten-day culture period, the annular body was moving up and down normally.

The sugar consumption of the cells was measured using a GlucCell Handy Glucose Monitor (CESCO) to find out that the sugar consumption of the cells increased as the number of days increased, and that the cells were appropriately cultured by using the cell culture device of this example.

On Day 10, as many as 3.0 x 10⁹ ADSCs were collected from within the cell culture vessel. Whether the collected ADSCs retained a normal karyotype was analyzed. The collected ADSCs were fixed with Carnoy's solution, and then subjected to simple karyotyping (Q-band) (chromocenter Inc.). The result showed that the collected ADSCs retained a normal karyotype. It was clear that the cells cultured by using the cell culture device of this example maintained a normal karyotype.

That is, Example 2 showed that even when as many as 5000 sheets of nonwoven fabric sheets were placed inside, the accurate up-down movement of the annular body was ensured, and that it was possible to culture cells while reducing the influence of the magnetic force on the cultured cells.

### (Example 3)

Differentiation of the ADSCs of Examples 1 and 2 after mass culture and collection into adipocytes, chondrocytes, and osteocytes was induced. For the induction of differentiation, Human Mesenchymal Stem Cell Functional IDentification Kit from R&D System, Inc. was used. To each of 24 well culture plates, 10⁴ cells were attached. Then, for induction of differentiation into adipocytes, a differentiation medium obtained by adding ADipogenic Supplement and ITS Supplement attached to the kit to 50 ml of DMEM basal medium was used and the cells were cultured for 14 days. For induction of differentiation into chondrocytes, a differentiation medium obtained by adding ChonDrogenic Supplement and ITS Supplement attached to the kit to 50 ml of DMEM basal medium was used and the cells were cultured for 21 days. For induction of differentiation into osteocytes, a differentiation medium obtained by adding Osteogenic Supplement and ITS Supplement attached to the kit to 50 ml of DMEM basal medium was used and the cells were cultured for 21 days.

In order to identify adipocytes, chondrocytes, and osteocytes after the induction of differentiation, OilReD-O, Alcian Blue, and Alizarin ReD staining were performed after 4% paraformaldehyde fixation. As a result, the ADSCs after the mass culture retained stem cell properties and pluripotency and differentiated into adipocytes, chondrocytes, and osteocytes.

### INDUSTRIAL APPLICABILITY

The present invention can be used for cell culture.

### DESCRIPTION OF REFERENCE CHARACTERS

- 100: Cell Culture Vessel
- 120: Bottom
- 125: Bottom Protrusion
- 126: Bottom Recess
- 130: Space
- 200: Dish-Shaped Body
- 220: Through Hole
- 230: Dish Recess
- 210: First Magnetic Body
- 300: Annular Body
- 310: Second Magnetic Body
- 320: Lift Device
- 350: Housing
- 351: Columnar Hollow Portion
- 352: Widened Portion
- 360: Third Magnetic Body
- 370: Stopper
- 400: Storage
- 401: Bottom Peripheral Wall
- 402: Inner Peripheral Wall
- 403: Outer Peripheral Wall
- 700: Vibration Device
- 710: Vibrator
- 900: Cell Culture Device

## Claims

1. A cell culture device (900) comprising:
a cell culture vessel (100) that is a substantially cylindrical body having, at a lower end, a bottom (120) that is flat, and having a hollow space (130) to be filled with a scaffold for culturing cells;
a dish-shaped body (200) having, in a periphery, a plurality of first magnetic bodies (210) each made of a ferromagnetic material at equal intervals, the dish-shaped body (200) having a substantially disk shape with a diameter slightly smaller than a diameter of the substantially cylindrical body of the cell culture vessel (100) so as to be disposed horizontally in the hollow space (130) of the cell culture vessel (100) without contact with an inner wall of the cell culture vessel (100) ;
an annular body (300) having a plurality of second magnetic bodies (310) each made of a magnet corresponding to an associated one of the first magnetic bodies (210) of the dish-shaped body (200) to attract the first magnetic bodies (210) of the dish-shaped body (200) by a magnetic force, the annular body (300) having a substantially ring shape and positioned outside the cell culture vessel (100) to have the cell culture vessel (100) located inside a ring of the cell culture vessel (100), the annular body (300) being configured to move upward to cause the dish-shaped body (200) to move upward in conjunction with the annular body (300) due to the magnetic force, thereby pushing up the scaffold that fills the hollow space of the cell culture vessel (100) from below, and move downward to cause the dish-shaped body (200) to move downward in conjunction with the annular body (300) due to the magnetic force, thereby making the scaffold that fills the hollow space of the cell culture vessel (100) fall by gravity; and
a storage (400) made of a ferromagnetic material and configured to store the annular body (300) when the annular body moves downward below the bottom of the cell culture vessel (100), wherein the storage (400) includes a bottom peripheral wall (401) circumferentially surrounding a bottom of the annular body (300), an outer peripheral wall (403) provided outside the bottom peripheral wall (401) and circumferentially surrounding an outside of the annular body (300), and an inner peripheral wall (402) provided inside the bottom peripheral wall (401) and circumferentially surrounding an inside of the annular body (300).

2. The cell culture device (900) of claim 1, wherein
the annular body (300) includes a housing (350) in which each of the second magnetic bodies (310) is housed with a gap so as to be radially slidable, and a plurality of third magnetic bodies (360) each made of a ferromagnetic material and corresponding to an associated one of the second magnetic bodies (310), the third magnetic bodies (360) being provided on a radially outer side of the housing (350),
when the annular body (300) moves downward below the bottom of the cell culture vessel (100) and is stored in the storage (400), the second magnetic bodies (310) are attracted to the third magnetic bodies (360) due to the magnetic force to slide in the housing (350) and move radially outward, and
when the annular body (300) moves up and above the bottom of the cell culture vessel (100) to be aligned with the dish-shaped body (200), the second magnetic bodies (310) are attracted to the first magnetic bodies (210) due to the magnetic force to slide in the housing (35) and move radially inward.

3. The cell culture device (900) of claim 2, wherein
a mass of each of the three magnetic bodies is smaller than a mass of each of the first magnetic bodies (210).

4. The cell culture device (900) of claim 2 or 3, wherein
the housing (350) includes a columnar hollow portion (351) that extends in a radial direction of the annular body (300) and a widened portion (352) positioned radially outside the columnar hollow portion (351), the columnar hollow portion (351) restricting a motion of the second magnetic bodies (310) other than sliding in a radial direction of each of the second magnetic bodies (310) due to close contact between an inner surface of the columnar hollow portion (351) and an outer surface of each of the second magnetic bodies (310).

5. The cell culture device (900) of claim 4, wherein
each of the second magnetic bodies (310) has a stopper (370) at an outer end in the radial direction of the annular body (300), the stopper (370) being positioned in the widened portion (352),
the widened portion (352) has a larger cross-sectional area perpendicular to the radial direction of the annular body (300) than the stopper (370), and the stopper (370) has a larger cross-sectional area perpendicular to the radial direction of the annular body (300) than the columnar hollow portion (351).

6. The cell culture device (900) of any one of claims 1 to 5, wherein
the scaffold that fills the cell culture vessel (100) is an aggregate of a plurality of small pieces supporting cells.

7. The cell culture device (900) of any one of claims 1 to 6, wherein
the cells to be cultured are mesenchymal stem cells.

## Patentansprüche

1. Zellkulturvorrichtung (900), umfassend:
ein Zellkulturgefäß (100), das einen im Wesentlichen zylindrischen Körper ist, der an einem unteren Ende einen flachen Boden (120) aufweist und einen Hohlraum (130) aufweist, der mit einem Gerüst zur Kultivierung von Zellen gefüllt werden kann;
einen schalenförmigen Körper (200), der am Umfang eine Vielzahl von ersten magnetischen Körpern (210) aufweist, die jeweils aus einem ferromagnetischen Material in gleichen Abständen bestehen, wobei der schalenförmige Körper (200) eine im Wesentlichen scheibenförmige Gestalt mit einem Durchmesser aufweist, der geringfügig kleiner ist als der Durchmesser des im Wesentlichen zylindrischen Körpers des Zellkulturgefäßes (100), sodass er horizontal im Hohlraum (130) des Zellkulturgefäßes (100) angeordnet werden kann, ohne mit der Innenwand des Zellkulturgefäßes (100) in Kontakt zu gelangen;
einen ringförmigen Körper (300), der eine Vielzahl von zweiten magnetischen Körpern (310) aufweist, von denen jeder aus einem Magneten besteht, der einem zugeordneten der ersten magnetischen Körper (210) des schalenförmigen Körpers (200) entspricht, um die ersten magnetischen Körper (210) des schalenförmigen Körpers (200) durch eine magnetische Kraft anzuziehen, wobei der ringförmige Körper (300) eine im Wesentlichen ringförmige Gestalt hat und außerhalb des Zellkulturgefäßes (100) positioniert ist, so dass sich das Zellkulturgefäß (100) innerhalb eines Rings des Zellkulturgefäßes (100) befindet, wobei der ringförmige Körper (300) so konfiguriert ist, dass er sich nach oben bewegt, um den schalenförmigen Körper (200) aufgrund der magnetischen Kraft in Verbindung mit dem ringförmigen Körper (300) nach oben zu bewegen, wodurch das Gerüst, das den Hohlraum des Zellkulturgefäßes (100) ausfüllt, von unten nach oben gedrückt wird, und sich nach unten zu bewegen, um zu bewirken, dass sich der schalenförmige Körper (200) aufgrund der magnetischen Kraft in Verbindung mit dem ringförmigen Körper (300) nach unten bewegt, wodurch das Gerüst, das den Hohlraum des Zellkulturgefäßes (100) ausfüllt, durch die Schwerkraft nach unten fällt; und
eine Aufbewahrungseinheit (400), die aus einem ferromagnetischen Material besteht und so konfiguriert ist, dass sie den ringförmigen Körper (300) aufnimmt, wenn sich der ringförmige Körper unterhalb des Bodens des Zellkulturgefäßes (100) nach unten bewegt, wobei die Aufbewahrungseinheit (400) eine untere Umfangswand (401), die den Boden des ringförmigen Körpers (300) umlaufend umgibt, eine äußere Umfangswand (403), die außerhalb der unteren Umfangswand (401) vorgesehen ist und die Außenseite des ringförmigen Körpers (300) umlaufend umgibt, sowie eine innere Umfangswand (402) enthält, die innerhalb der unteren Umfangswand (401) vorgesehen ist und die Innenseite des ringförmigen Körpers (300) umlaufend umgibt.

2. Zellkulturvorrichtung (900) nach Anspruch 1, wobei
der ringförmige Körper (300) ein Gehäuse (350) enthält, in dem jeder der zweiten magnetischen Körper (310) mit einem Spalt so untergebracht ist, dass er radial verschiebbar ist, sowie eine Vielzahl von dritten magnetischen Körpern (360), die jeweils aus einem ferromagnetischen Material bestehen und einem zugeordneten zweiten magnetischen Körper (310) entsprechen, wobei die dritten magnetischen Körper (360) an der radial äußeren Seite des Gehäuses (350) vorgesehen sind,
wenn sich der ringförmige Körper (300) nach unten unter den Boden des Zellkulturgefäßes (100) bewegt und in der Aufbewahrungseinheit (400) aufbewahrt wird, werden die zweiten magnetischen Körper (310) aufgrund der magnetischen Kraft zu den dritten magnetischen Körpern (360) angezogen, um im Gehäuse (350) zu gleiten und sich radial nach außen zu bewegen, und
wenn sich der ringförmige Körper (300) nach oben und über den Boden des Zellkulturgefäßes (100) bewegt, um mit dem schalenförmigen Körper (200) ausgerichtet zu werden, die zweiten magnetischen Körper (310 aufgrund der magnetischen Kraft zu den ersten magnetischen Körpern (210) angezogen werden, um im Gehäuse (35) zu gleiten und sich radial nach innen zu bewegen.

3. Zellkulturvorrichtung (900) nach Anspruch 2, wobei
eine Masse jedes der drei magnetischen Körper kleiner ist als eine Masse jedes der ersten magnetischen Körper (210).

4. Zellkulturvorrichtung (900) nach Anspruch 2 oder 3, wobei
das Gehäuse (350) einen säulenförmigen, hohlen Abschnitt (351), der sich in Radialrichtung des ringförmigen Körpers (300) erstreckt, sowie einen verbreiterten Abschnitt (352) enthält, der radial außerhalb des säulenförmigen, hohlen Abschnitts (351) positioniert ist, wobei der säulenförmige, hohle Abschnitt (351) eine Bewegung der zweiten magnetischen Körper (310), außer dem Gleiten in Radialrichtung jedes der zweiten magnetischen Körper (310), aufgrund des engen Kontakts zwischen einer Innenfläche des säulenförmigen, hohlen Abschnitts (351) und einer Außenfläche jedes der zweiten magnetischen Körper (310) einschränkt.

5. Zellkulturvorrichtung (900) nach Anspruch 4, wobei
jeder der zweiten magnetischen Körper (310) an einem äußeren Ende in radialer Richtung des ringförmigen Körpers (300) einen Anschlag (370) aufweist, wobei sich der Anschlag (370) im verbreiterten Abschnitt (352) positioniert ist,
der verbreiterte Abschnitt (352) eine größere Querschnittsfläche senkrecht zur Radialrichtung des ringförmigen Körpers (300) aufweist als der Anschlag (370), und der Anschlag (370) eine größere Querschnittsfläche senkrecht zur Radialrichtung des ringförmigen Körpers (300) aufweist als der säulenförmige, hohle Abschnitt (351).

6. Zellkulturvorrichtung (900) nach einem der Ansprüche 1 bis 5, wobei
das Gerüst, das das Zellkulturgefäß (100) ausfüllt, ein Aggregat aus einer Vielzahl von kleinen, die Zellen tragenden Teilen ist.

7. Zellkulturvorrichtung (900) nach einem der Ansprüche 1 bis 6, wobei
die zu kultivierenden Zellen mesenchymale Stammzellen sind.

## Revendications

1. Dispositif de culture cellulaire (900) comprenant :
un récipient de culture cellulaire (100) qui est un corps sensiblement cylindrique ayant, au niveau d'une extrémité inférieure, un fond (120) qui est plat, et ayant un espace creux (130) pour être rempli d'un échafaudage pour la culture de cellules ;
un corps en forme de coupelle (200) ayant, dans une périphérie, une pluralité de premiers corps magnétiques (210) réalisés chacun en un matériau ferromagnétique à intervalles égaux, le corps en forme de coupelle (200) ayant une forme sensiblement de disque avec un diamètre légèrement inférieur à un diamètre du corps sensiblement cylindrique du récipient de culture cellulaire (100) de manière à être disposé horizontalement dans l'espace creux (130) du récipient de culture cellulaire (100) sans contact avec une paroi interne du récipient de culture cellulaire (100) ;
un corps annulaire (300) ayant une pluralité de deuxièmes corps magnétiques (310) réalisés chacun en un aimant correspondant à un corps associé des premiers corps magnétiques (210) du corps en forme de coupelle (200) pour attirer les premiers corps magnétiques (210) du corps en forme de coupelle (200) par une force magnétique, le corps annulaire (300) ayant une forme sensiblement annulaire et étant positionné à l'extérieur du récipient de culture cellulaire (100) pour que le récipient de culture cellulaire (100) soit situé à l'intérieur d'un anneau du récipient de culture cellulaire (100), le corps annulaire (300) étant configuré pour se déplacer vers le haut afin d'amener le corps en forme de coupelle (200) à se déplacer vers le haut conjointement avec le corps annulaire (300) en raison de la force magnétique, poussant ainsi vers le haut l'échafaudage qui remplit l'espace creux du récipient de culture cellulaire (100) par le dessous, et se déplacer vers le bas afin d'amener le corps en forme de coupelle (200) à se déplacer vers le bas conjointement avec le corps annulaire (300) en raison de la force magnétique, permettant ainsi à l'échafaudage qui remplit l'espace creux du récipient de culture cellulaire (100) de tomber par gravité ; et
un élément de stockage (400) réalisé en un matériau ferromagnétique et configuré pour stocker le corps annulaire (300) lorsque le corps annulaire se déplace vers le bas en dessous du fond du récipient de culture cellulaire (100), où l'élément de stockage (400) comprend une paroi périphérique de fond (401) entourant de manière circonférentielle un fond du corps annulaire (300), une paroi périphérique externe (403) prévue à l'extérieur de la paroi périphérique de fond (401) et entourant de manière circonférentielle l'extérieur du corps annulaire (300), et une paroi périphérique interne (402) prévue à l'intérieur de la paroi périphérique de fond (401) et entourant de manière circonférentielle l'intérieur du corps annulaire (300).

2. Dispositif de culture cellulaire (900) selon la revendication 1, dans lequel
le corps annulaire (300) comprend un logement (350) dans lequel chacun des deuxièmes corps magnétiques (310) est logé avec un jeu de manière à pouvoir coulisser radialement, et une pluralité de troisièmes corps magnétiques (360) réalisés chacun en un matériau ferromagnétique et correspondant à un corps associé des deuxièmes corps magnétiques (310), les troisièmes corps magnétiques (360) étant prévus sur un côté radialement externe du logement (350),
lorsque le corps annulaire (300) se déplace vers le bas en dessous du fond du récipient de culture cellulaire (100) et est stocké dans l'élément de stockage (400), les deuxièmes corps magnétiques (310) sont attirés par les troisièmes corps magnétiques (360) en raison de la force magnétique pour coulisser dans le logement (350) et se déplacer radialement vers l'extérieur, et
lorsque le corps annulaire (300) se déplace vers le haut et au-dessus du fond du récipient de culture cellulaire (100) pour être aligné avec le corps en forme de coupelle (200), les deuxièmes corps magnétiques (310) sont attirés par les premiers corps magnétiques (210) en raison de la force magnétique pour coulisser dans le logement (35) et se déplacer radialement vers l'intérieur.

3. Dispositif de culture cellulaire (900) selon la revendication 2, dans lequel
une masse de chacun des troisièmes corps magnétiques est inférieure à une masse de chacun des premiers corps magnétiques (210).

4. Dispositif de culture cellulaire (900) selon la revendication 2 ou 3, dans lequel
le logement (350) comprend une partie creuse en forme de colonne (351) qui s'étend dans une direction radiale du corps annulaire (300) et une partie élargie (352) positionnée radialement à l'extérieur de la partie creuse en forme de colonne (351), la partie creuse en forme de colonne (351) limitant un mouvement des deuxièmes corps magnétiques (310) autre qu'un coulissement dans une direction radiale de chacun des deuxièmes corps magnétiques (310) en raison d'un contact étroit entre une surface interne de la partie creuse en forme de colonne (351) et une surface externe de chacun des deuxièmes corps magnétiques (310).

5. Dispositif de culture cellulaire (900) selon la revendication 4, dans lequel
chacun des deuxièmes corps magnétiques (310) a une butée (370) au niveau d'une extrémité externe dans la direction radiale du corps annulaire (300), la butée (370) étant positionnée dans la partie élargie (352),
la partie élargie (352) a une surface de section transversale, perpendiculaire à la direction radiale du corps annulaire (300), supérieure à celle de la butée (370), et la butée (370) a une surface de section transversale, perpendiculaire à la direction radiale du corps annulaire (300), supérieure à celle de la partie creuse en forme de colonne (351).

6. Dispositif de culture cellulaire (900) selon l'une quelconque des revendications 1 à 5, dans lequel
l'échafaudage qui remplit le récipient de culture cellulaire (100) est un agrégat d'une pluralité de petits morceaux supportant des cellules.

7. Dispositif de culture cellulaire (900) selon l'une quelconque des revendications 1 à 6, dans lequel
les cellules à mettre en culture sont des cellules souches mésenchymateuses.
